# EUROPEAN PATENT APPLICATION

(11) **EP 2 813 196 A1**
(43) Date of publication of application: **17.12.2014**
(21) Application number: 13186024.9
(22) Date of filing: 25.09.2013
(51) Int. Cl.: A61F 7/02, A61F 7/10

(54) **Method of manufacturing disposable cold pack and related disposable cold pack containing urea and ammonium chloride**

(30) Priority: 14.06.2013 US 201313918229
(71) Applicant: Rapid Aid Corp., Mississauga Ontario L5L 5Z3 (CA)
(72) Inventor: Whitely, Jeffrey, Mississauga, Ontario L5L 5Z3 (CA)
(74) Representative: Walker, Ross Thomson

(57) **Abstract**

Plastic sheet can be heat-sealed to form a water-tight envelope having an open top. A water-tight frangible container can be loaded into the envelope through the open top. The water-tight frangible container can contain a predetermined amount of water. A predetermined amount of urea can be loaded into the envelope through the open top. Separate from the loading of the urea, a predetermined amount of ammonium chloride can be loaded into the envelope through the open top. The relative proportions of the predetermined amount of urea, the predetermined amount of ammonium chloride, and the predetermined amount of water can be selected to provide a target cooling effect to a portion of a body to which cooling is to be applied. The open top of the envelope can be heat-sealed to form a water-tight outer pack.

## Description

### Related Applications

This application claims priority to US patent application no. 13/918,229.

### Field

The present invention relates to cold packs.

### Background

Reusable cold packs are known, however, these are not used in many clinical situations because they require freezing and because of sterility concerns and the need to reduce the spread of disease.

Certain kinds of disposable cold packs are also known, however, these suffer from drawbacks including poor manufacturability, design complexity, and high unit cost.

In addition, certain materials used in disposable cold packs, such as ammonium nitrate, may be controlled or regulated and therefore difficult to obtain and potentially hazardous to store in quantities suitable for mass production of disposable cold packs.

Therefore, the prior art suffers for lack of a disposable cold pack that is readily manufacturable in large quantities and convenient and safe to use in various clinical situations.

### Summary

The present invention relates to disposable cold packs for cold therapy and methods of manufacturing same. Such disposable cold packs can include a mixture of urea and ammonium chloride.

According to one aspect of the present invention, a method of manufacturing a disposable cold pack for cold therapy includes heat-sealing plastic sheet to form a water-tight envelope having an open top and loading a predetermined amount of urea into the envelope through the open top. The method further includes, separate from the loading of the urea, loading a predetermined amount of ammonium chloride into the envelope through the open top. The method further includes loading a water-tight frangible container into the envelope through the open top. The water-tight frangible container contains a predetermined amount of water. The relative proportions of the predetermined amount of urea, the predetermined amount of ammonium chloride, and the predetermined amount of water are selected to provide a target cooling effect to a portion of a body to which cooling is applied. The method further includes heat-sealing the open top of the envelope to form a water-tight outer pack.

According to another aspect of the present invention, a disposable cold pack for providing cold therapy to a portion of a body includes an outer pack formed of a water-tight sealed envelope. The outer pack has an outer surface configured for placement on skin or clothing covering the portion of the body. The cold pack further includes a mixture of urea and ammonium chloride contained inside the outer pack. The cold pack further includes a water-tight frangible container disposed inside the outer pack. The water-tight frangible container contains water, and is configured to release the water into the inside of the outer pack in response to an external force applied to the outer pack. The relative proportions of the urea, the ammonium chloride, and the water are selected to provide a target cooling effect to the portion of the body.

According to another aspect of the present invention, a disposable cold pack for providing cold therapy to a portion of a body includes an outer pack formed of a water-tight sealed envelope formed of at least one layer of plastic sheet that is heat-sealed at one or more heat-sealed seams. The plastic sheet has a fibre texture at an outer surface of the outer pack to configure the outer pack for placement in direct contact with skin of the portion of the body. The cold pack further includes a mixture of urea and ammonium chloride contained inside the outer pack. The mixture does not contain a substantial amount of ammonium nitrate. The cold pack further includes a water-tight frangible container disposed inside the outer pack. The water-tight frangible container includes a heat-sealed plastic bag containing water, and is configured to rupture and release the water into the inside of the outer pack in response to an external force applied to the outer pack. The relative proportions of the urea, the ammonium chloride, and the water are selected to provide a target cooling effect to the portion of the body. The relative proportions are between about 32 - 38% urea, between about 7 - 16.5% ammonium chloride, and between about 45.5 - 61% water, by weight.

### Brief Description of the Figures

The drawings illustrate, by way of example only, embodiments of the present invention.

FIG. 1 is a perspective view of a cold pack according to an embodiment of the present invention.

FIG. 2 is a cross-sectional view of the cold pack of FIG. 1.

FIG. 3 is a perspective view of a cold pack with a strap according to another embodiment of the present invention.

FIGs. 4a - f are schematic diagrams of a method of manufacturing the cold pack FIG. 1.

FIGs. 4g - h are schematic diagrams of steps for attaching the strap to the cold pack of FIG. 3.

FIG. 5 is a close-up view of the attachment of the strap to the cold pack of FIG. 3.

FIG. 6 is a diagram of stock side-sealed material that can be cut to length to form envelopes of various lengths.

FIG. 7 is a diagram of another embodiment of a cold pack having a strap connected to an end opposite of the end that is used for filling the cold pack.

FIG. 8 is a diagram of a cold pack shaped and sized to fit a female perineum according to another embodiment of the present invention.

### Detailed Description

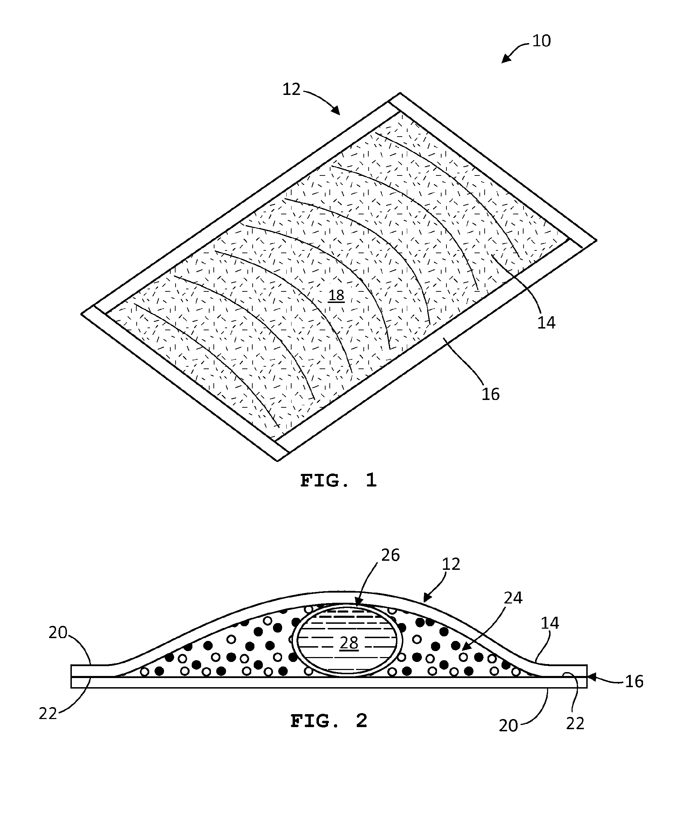

FIG. 1 shows a disposable cold pack 10 for providing cold therapy. The cold pack 10 can be applied to a portion of a person's body, such as an arm, leg, neck, abdomen, etc. The cold pack 10 contains a mixture of urea (CH₄N₂O) and ammonium chloride (NH₄Cl), which reacts spontaneously and endothermically with water, so as to cool the portion of the body for therapeutic effect. The cold pack 10 may be used to reduce swelling or discomfort associated with injury, and may find other uses as well. The cold pack 10 is disposable, in that once the endothermic reaction is complete, the cold pack 10 is not reusable and can be discarded. The cold pack 10 may be referred to as an instant cold pack because the mixture is selected so that the endothermic reaction occurs quickly.

The reaction of urea and water does not achieve temperatures cold enough for many therapeutic applications. Hence, according to the invention, ammonium chloride is included with the urea to reduce the temperature further. This provides the benefit of temperatures comparable to those that can be attained when using ammonium nitrate, but without the need to use ammonium nitrate and be limited by its distinct disadvantages.

The cold pack 10 includes an outer pack 12 formed of a water-tight sealed envelope 14. The envelope 14 may be formed of at least one layer of plastic sheet, which can be heat-sealed at one or more seams 16 that define the outer perimeter of the envelope 14. In the embodiment depicted, all four edges of the rectangular envelope 14 have heat-sealed seams 16. In other embodiments, three edges of the rectangular envelope 14 have heat-sealed seams 16, with the fourth edge being formed by a fold of a single sheet of plastic.

The plastic sheet may be of any suitable polymer, such as polyethylene, polyester, polypropylene, nylon, polyvinyl chloride, and combinations of these materials, such as laminates of multiple layers of these materials, as well laminates of such materials further including paper. The plastic sheet material may be selected to be free of latex and other allergenic materials.

The plastic sheet can have a fibre texture at an outer surface 18 of the outer pack 12 to configure the outer pack 12 for placement in direct contact with skin of the portion of the body. The fibre texture provides an amount of thermal insulation to disperse the cooling effect of the cold pack 10 and prevent the skin for becoming too cold. The fibre texture can reduce or eliminate the need to use a wrap or towel around the cold pack, and therefore reduce the time it takes to apply the cold pack, which can be important in emergency situations. The cold pack 10 can be used against clothes as well, and the fibre texture does not limit potential modes of application of the cold pack 10.

The plastic sheet can have the fibre texture on one side 20 and bare plastic on the other side 22. The envelope 14 is formed with the fibre texture on the outside surface 18 and the bare plastic on opposite, inside surfaces, at 22, which undergo the heat-sealing.

The fibre texture can be formed by filaments or fibers that are integral to the plastic sheet and made of the same material as the plastic sheet. Alternatively, the fibre texture can be formed separate from the plastic sheet, such as a fabric weave, paper, or similar, that is then joined to the plastic sheet, by adhesive, heat-sealing, etc.

With reference to FIG. 2, the outer pack 12 contains a mixture 24 of urea and ammonium chloride. The mixture 24 does not contain a substantial amount of ammonium nitrate. A substantial amount of ammonium nitrate can be defined as, for example, an amount that is required by regulation to be indicated on a label of the cold pack 10. Another example of a substantial amount of ammonium nitrate is an amount that can be removed from the cold pack 10 using only basic tools and used for another purpose. Because ammonium nitrate is relatively volatile, can be explosive under certain conditions, and is regulated in many jurisdictions, it can be advantageous to omit such material from the cold pack. In some embodiments, small or trace amounts of ammonium nitrate can be present. In other embodiments, no ammonium nitrate is present in the cold pack 10.

Also disposed inside the outer pack 12 is a water-tight frangible container 26, which can include a heat-sealed plastic bag, and glass ampoule, or the like. The frangible container 26 contains water 28 and is configured to rupture and release the water 28 into the inside of the outer pack 12 in response to an external force applied to the outer pack 12. When the water is released and mixes with the mixture 24 of urea and ammonium chloride, an endothermic reaction takes place, thereby fulfilling the function of the disposable cold pack 10.

In some embodiments, the water-tight frangible container 26 is a plastic bag that is shaped and sized to rupture when the outer pack 12 is folded over itself about substantially any folding axis. To achieve this, the water-tight frangible container 26 can be rectangular in shape and sized to cover about more than half of the area within the outer pack 12, as measured parallel to the plastic sheet forming the envelope 14 of the outer pack 12.

It is advantageous that both urea and ammonium chloride are highly soluble in water and non-toxic. The relative proportions of urea, ammonium chloride, and water can be selected to tailor the cooling effect for the specific application of the cold pack and the material used for the outer pack, so that sufficient, but not too much, cooling is provided. Further, the relative proportions of urea, ammonium chloride, and water can be selected to take into account the expected initial starting temperature of the cold pack, just prior to activation.

The relative proportions of the urea, the ammonium chloride, and the water can be selected to provide a target cooling effect to the portion of the body to which the cold pack 10 is applied. The relative proportions can be between about 32 - 38% urea, between about 7 - 16.5% ammonium chloride, and between about 45.5 - 61% water, by weight.

In some embodiments, the relative proportions are between about 32 - 36% urea, between about 12.5 - 16.5% ammonium chloride, and between about 47.5 - 55.5% water, by weight. These proportions are suitable for general use of the cold pack, particularly when the fibre texture is provided to the outside surface 18.

In some embodiments, the outer pack 12 is sized and shaped to fit a female perineum, and the relative proportions are between about 34 - 38% urea, between about 7 - 11% ammonium chloride, and between about 51 - 59% water, by weight. Such proportions are suitable when the fibre texture is provided to the outside surface 18. Furthermore, such proportions can prevent the cold pack from becoming too cool and causing cold injury during postpartum application, while simultaneously reducing or eliminating the need to wrap the cold pack in a towel or other temperature buffer.

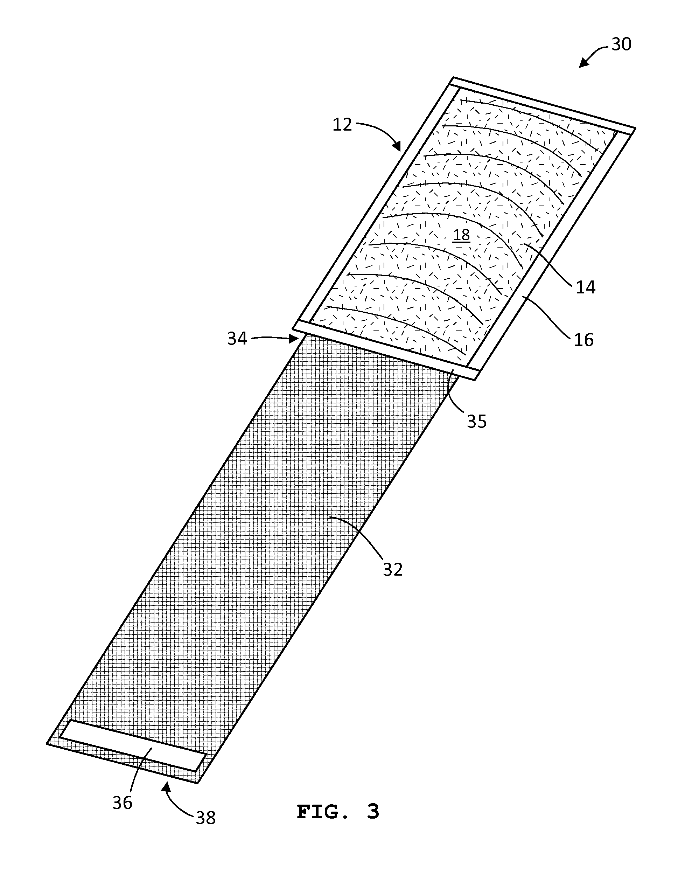

FIG. 3 shows a disposable cold pack 30 having a strap. The cold pack 30 is similar to the cold pack 10. Like reference numerals designate like elements, and the description for the cold pack 10 can be referenced.

The cold pack 30 includes a strap 32 having a fixed end 34 heat-bonded between overlapping portions of the plastic sheet, which form the envelope 14, at one of the heat-sealed seams 35. The strap 32 can be made of a material such as gauze, can be permeable to air, and can have some elastic stretch. The strap 32 has a length selected to wrap around the portion of the body and over the outer pack 12, so as to secure the cold pack 30 to the area of the body in need of cooling. The strap 32 includes a fastener 36 at a free end 38 opposite the fixed end 34. The fastener 36 is configured to engage the material of the strap 32. The fastener 36 can include a hook side of a hook-and-loop fastener (e.g., Velcro TM).

Further embodiments and implementation details of thermotherapeutic pads can be found in US patent application publication 2012/0004713.

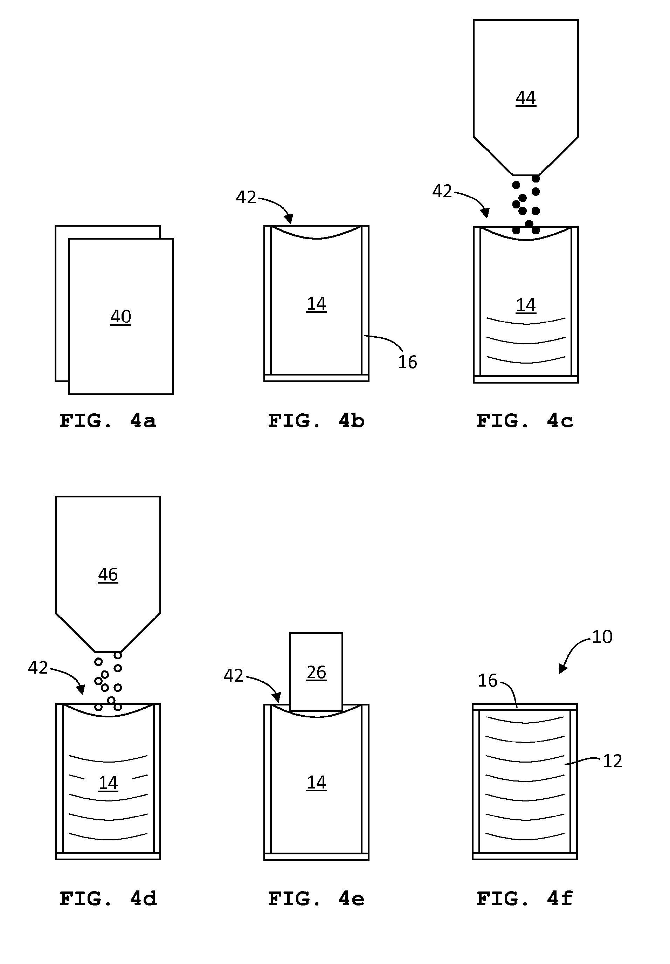

FIGs. 4a - 4f illustrate a method of manufacturing a disposable cold pack, such as the cold pack 10 discussed above. Like reference numerals designate like elements, and the description for the cold pack 10 can be referenced. The method is schematically illustrated, and equipment such as a rotary band sealer, an impulse sealer, a vertical form fill sealing machine, a horizontal form fill sealing machine, and similar may be used to carry out one, several, or all of the steps described.

As shown in FIG. 4a, one or more pieces of plastic sheet 40 are provided. The pieces of plastic sheet 40 can be cut from stock sheet to a suitable size and shape. In some embodiments, the plastic sheet is cut from stock to have a size and shape that provides an outer pack with a size and shape selected to fit a typical female perineum region.

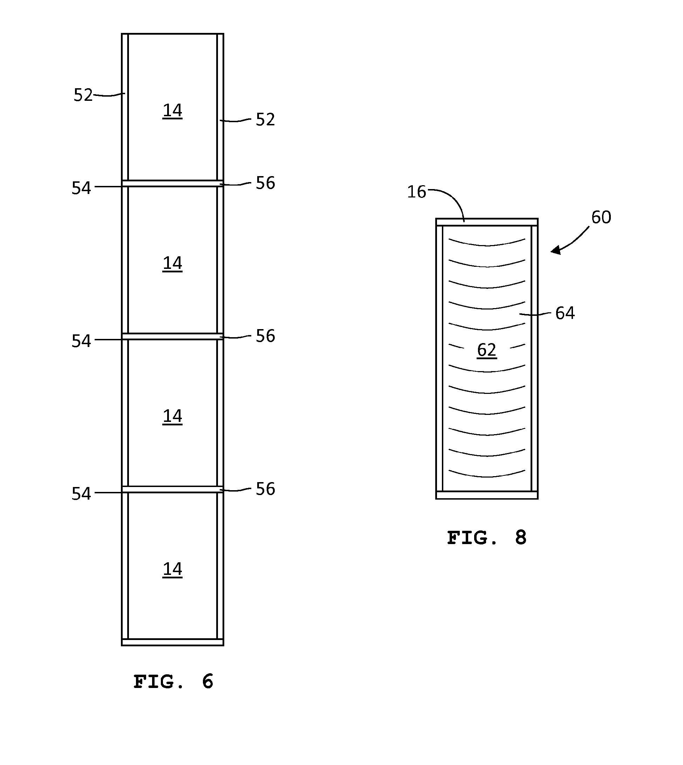

Next, as shown in FIG. 4b, the plastic sheet 40 is heat-sealed to provide heat-sealed seams 16 to three edges and form a water-tight envelope 14 having an open top 42. In other embodiments, as shown in FIG. 6, two opposite side seams 52 are heat-sealed for a length of plastic sheet that is longer than the envelope 14, and the side-sealed plastic sheet is subsequently cut to length, at 54, before or after the bottom seam 56 is heat-sealed. This can advantageously allow use of the same roll of stock sheet to form cold packs of various different lengths.

Subsequently, as shown in FIG. 4c, a predetermined amount of urea is loaded into the envelope 14 through the open top 42. A hopper 44 can be used to load the urea. The hopper 44 can be configured to dispense the predetermined amount of urea.

Further, as shown in FIG. 4d, a predetermined amount of ammonium chloride is loaded into the envelope 14 through the open top 42. Another hopper 46 can be used to load the ammonium chloride. The hopper 46 can be configured to dispense the predetermined amount of ammonium chloride.

Then, as shown in FIG. 4e, a water-tight frangible container 26 is loaded into the envelope 14 through the open top 42. The water-tight frangible container 26 contains a predetermined amount of water. The water-tight frangible container 26 can be formed by loading an open end of a plastic bag with the predetermined amount of water and then heat-sealing the open end of the plastic bag. The same heat-sealing equipment can be used for the water-tight frangible container 26 and the envelope seams 16.

In this embodiment, the ammonium chloride is loaded separately from the loading of the urea. This allows the process to be flexible, in contrast to batch mixing, so as to allow different proportions of ammonium chloride and urea to be mixed, as needed. The order in which the urea and the ammonium chloride are loaded can be varied, and it may be easier, or provide for faster manufacture, to load the water-tight frangible container 26 after loading the urea and the ammonium chloride.

In other embodiments, the ammonium chloride and urea are loaded from separate hoppers into a mixing apparatus before being loaded together into the envelope 14. A cup filler or similar apparatus may be used to achieve this.

The relative proportions of the predetermined amount of urea, the predetermined amount of ammonium chloride, and the predetermined amount of water in the water-tight frangible container 26 are selected to provide a target cooling effect. Example proportions are discussed elsewhere herein.

Lastly, as shown in FIG. 4f, the open top 42 of the envelope 14 is heat-sealed, at seam 16, to form a water-tight outer pack 12 and complete manufacture of the disposable cold pack 10.

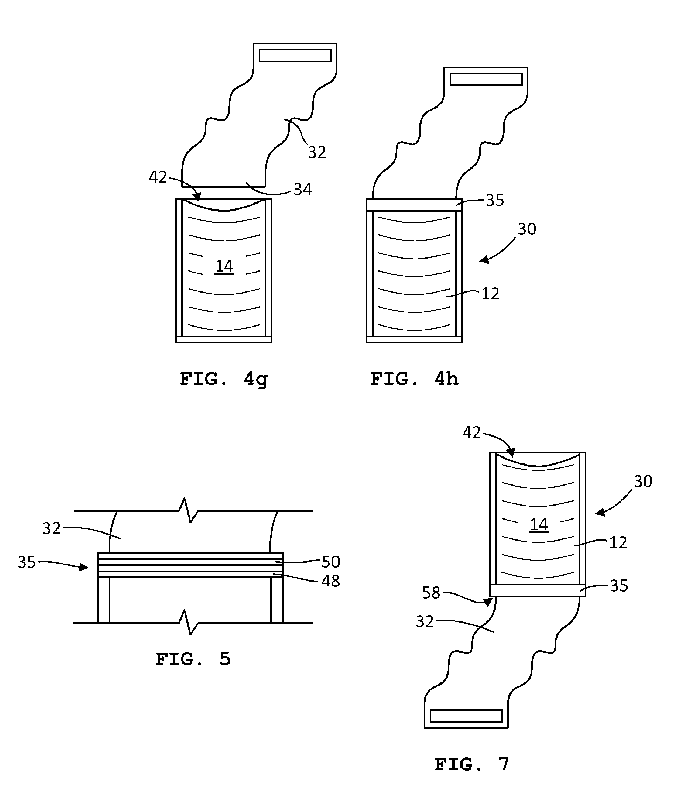

FIGs. 4g - 4h illustrate steps of a method of manufacturing a disposable cold pack having a strap, such as the cold pack 30 discussed above. Like reference numerals designate like elements, and the description for the cold packs 10, 30 can be referenced.

As shown in FIG. 4g, an end 34 of a strap 32 is inserted into the open top 42 of the envelope 14. This can be performed after the envelope 14 is filled with the water container 26, the urea, and the ammonium chloride (i.e., after the step shown in FIG. 4e).

Then, as shown in FIG. 4h, the open top 42 of the envelope 14 is heat-sealed, at seam 35, to affix the strap 32 to the outer pack 12 and provide a water-tight seal to the outer pack 12 to complete the instant cold pack 30 (i.e., instead of the step shown in FIG. 4f).

The heat-sealed seam 35 can be a single seam, such that the heat-sealing action of the step in FIG. 4h simultaneously affixes the strap 32 to the envelope 14 and seals the envelope 14 to form the water-tight outer pack 12. This can advantageously reduce the time required to manufacture the cold pack 30.

Alternatively, as shown in FIG. 5, the heat-sealed seam 35 can be a composite seam that includes two separate heat-sealed seams 48 and 50. The inner heat-sealed seam 48 does not affix the strap 32 to the envelope 14, and serves to provide a water-tight seal to the outer pack 12. In some embodiments, the seams 48, 50 are made at about the same time and the end 34 of the strap 32 can be prevented from being inserted too far into the envelope 14, so as to allow forming of the inner heat-sealed seam 48 without interference from the strap 32. In other embodiments, the strap is affixed at seam 50 at the top or bottom of the envelope 14 after all sealing seams, such as seam 48, have been made. This can be accomplished by leaving an unsealed outside margin of plastic sheet material when making the water-tight seal. The outer heat-sealed seam 50 affixes the strap 32 to the envelope 14 and is not relied upon to provide water-tightness. Use of this composite seam can advantageously prevent rupture of the outer pack 12 due to stresses on the strap 32, and help to ensure water-tightness of the outer pack 12. The same heat-sealing equipment can be used for the two seams 48, 50.

In another embodiment, as shown in FIG. 7, the strap 32 is attached to the bottom end 58 of the envelope 14 before or after the envelope 14 is filled with the water container 26, the urea, and the ammonium chloride at the top 42. A single heat-sealed seam or a composite heat-sealed seam can be used to attach the strap 32. In some embodiments, the strap 32 is attached at a seam 50 to the bottom of the envelope 14 after the envelope 14 is sealed completely closed, as this can allow for faster manufacture.

FIG. 8 shows a cold pack 60 according to another embodiment. Like reference numerals designate like elements, and the description for the cold packs 10, 30 can be referenced.

The cold pack 60 is made with an envelope 62 cut from plastic sheet stock to provide for an outer pack 64 with a size and shape selected to fit a female perineum. With reference to FIG. 6, it should be noted that the envelopes 62 and 14 can be selected to have the same width and therefore can be cut from the same length of side-sealed plastic sheet material. For further understanding of the features and aspects of the cold pack 60, the description of the cold pack 10 can be referenced.

It can be seen from the above, that the present invention provides a disposable cold pack that is readily manufacturable in large quantities and convenient and safe to use in various clinical situations.

While the foregoing provides certain non-limiting example embodiments, it should be understood that combinations, subsets, and variations of the foregoing are contemplated. The monopoly sought is defined by the claims.

## Claims

1. A method of manufacturing a disposable cold pack for cold therapy, the method comprising:
heat-sealing plastic sheet to form a water-tight envelope having an open top;
loading a predetermined amount of urea into the envelope through the open top;
separate from the loading of the urea, loading a predetermined amount of ammonium chloride into the envelope through the open top;
loading a water-tight frangible container into the envelope through the open top, the water-tight frangible container containing a predetermined amount of water, relative proportions of the predetermined amount of urea, the predetermined amount of ammonium chloride, and the predetermined amount of water selected to provide a target cooling effect to a portion of a body to which cooling is applied; and
heat-sealing the open top of the envelope to form a water-tight outer pack.

2. The method of claim 1, wherein the plastic sheet has a fibre texture on one side and bare plastic on an opposite side, and the envelope is formed with the fibre texture on an outside surface and the bare plastic on an inside surface that undergoes the heat-sealing.

3. The method of claim 1 or 2, wherein the relative proportions are any one of:
between about 32 - 36% urea, between about 12.5 - 16.5% ammonium chloride, and between about 47.5 - 55.5% water, by weight; and between about 34 - 38% urea, between about 7 - 11% ammonium chloride, and between about 51 - 59% water, by weight.

4. The method of any one of claims 1 to 3, further comprising cutting the plastic sheet from stock to have a size and shape that provides the outer pack with a size and shape selected to fit a female perineum.

5. The method of any one of claims 1 to 4, wherein the envelope is not loaded with a substantial amount of ammonium nitrate.

6. The method of any one of claims 1 to 5, further comprising:
inserting an end of a strap between plastic sheet of the envelope after the open top of the envelope has been heat-sealed, the strap having a fastener at a free end opposite the inserted end; and
heat-bonding the strap to the envelope.

7. The method of any one of claims 1 to 5, further comprising inserting an end of a strap into the open top of the envelope, the strap having a fastener at a free end opposite the inserted end, wherein heat-sealing the open top of the envelope affixes the strap to the outer pack and provides a water-tight seal to the outer pack.

8. The method of any one of claims 1 to 7, further comprising forming the water-tight frangible container by loading an open end of a plastic bag with the predetermined amount of water and heat-sealing the open end of the plastic bag.

9. A disposable cold pack for providing cold therapy to a portion of a body, the cold pack comprising:
an outer pack formed of a water-tight sealed envelope, the outer pack having an outer surface configured for placement on skin or clothing covering the portion of the body;
a mixture of urea and ammonium chloride contained inside the outer pack; and
a water-tight frangible container disposed inside the outer pack, the water-tight frangible container containing water, the water-tight frangible container configured to release the water into the inside of the outer pack in response to an external force applied to the outer pack;
relative proportions of the urea, the ammonium chloride, and the water selected to provide a target cooling effect to the portion of the body.

10. The disposable cold pack of claim 9, wherein the water-tight sealed envelope is made of plastic sheet having a fibre texture at the outer surface of the outer pack.

11. The disposable cold pack of claim 9 or 10, wherein the relative proportions are one of:
between about 32 - 36% urea, between about 12.5 - 16.5% ammonium chloride, and between about 47.5 - 55.5% water, by weight; and
between about 34 - 38% urea, between about 7 - 11% ammonium chloride, and between about 51 - 59% water, by weight.

12. The disposable cold pack of any one of claims 9 to 11, wherein the outer pack does not contain a substantial amount of ammonium nitrate.

13. The disposable cold pack of any one of claims 9 to 12, wherein the water-tight sealed envelope is formed of at least one layer of plastic sheet that is heat-sealed at one or more heat-sealed seams.

14. The disposable cold pack of claim 13, further comprising a strap having a fixed end heat-bonded between overlapping portions of the plastic sheet, the strap having a length selected to wrap around the portion of the body part and over the outer pack, the strap having a fastener at a free end opposite the fixed end, the fastener configured to engage a material of the strap.

15. The disposable cold pack of any one of claims 9 to 14, wherein the water-tight frangible container comprises a heat-sealed plastic bag.
